(19) **Europäisches Patentamt European Patent Office Office européen des brevets**

(11) **EP 3 186 628 B1**

(12) **EUROPEAN PATENT SPECIFICATION**

(45) Date of publication and mention
of the grant of the patent:
**31.01.2018 Bulletin 2018/05**

(51) Int Cl.:
***G01N 33/00*** *(2006.01)*

(21) Application number: **15759700.6**

(86) International application number:
**PCT/EP2015/069465**

(22) Date of filing: **25.08.2015**

(87) International publication number:
**WO 2016/030386 (03.03.2016 Gazette 2016/09)**

(54) **SENSOR SYSTEM AND SENSING METHOD**

SENSORSYSTEM UND MESSVERFAHREN

SYSTÈME DE CAPTEUR ET PROCÉDÉ DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2014 PCT/CN2014/085397**
**03.02.2015 EP 15153626**

(43) Date of publication of application:
**05.07.2017 Bulletin 2017/27**

(73) Proprietor: **Koninklijke Philips N.V.**
**5656 AE Eindhoven (NL)**

(72) Inventors:
• **KE, Rui**
**NL-5656 AE Eindhoven (NL)**
• **RONDA, Cornelis Reinder**
**NL-5656 AE Eindhoven (NL)**

(74) Representative: **Tassignon, Tom**
**Philips International B.V.**
**Intellectual Property & Standards**
**High Tech Campus 5**
**5656 AE Eindhoven (NL)**

(56) References cited:
**EP-A1- 0 764 331      EP-A1- 2 762 877**
**WO-A1-2013/119219      WO-A2-2013/008170**
**US-A1- 2012 229 287      US-A1- 2013 074 575**

**Description**

FIELD OF THE INVENTION

The invention relates to the sensing of substances in a fluid, for example measuring pollutant concentrations in air and to a method for sensing substances in a fluid.

BACKGROUND OF THE INVENTION

Airborne particle pollution, especially particle matter size less than 2.5 $\mu$m diameter range (named "PM2.5"), is a big concern for consumers, especially in countries which rapidly industrialize such as China.

As a consequence of increasing consumer empowerment, the demand for information about the air quality of living spaces is increasing. In countries such as China, excessive PM2.5 pollution has become a common problem in the last decade. This problem is also validated by continuous measurements in various Chinese cities. The data is publicly available and can be simultaneously monitored by mobile phone applications or through the web.

Availability of this data as well as continuous national and international media attention has created strong consumer awareness about the problem.

Official outdoor air quality standards define particle matter concentration as mass concentration per unit volume (e.g. $\mu$g/m$^3$). The average PM2.5 pollution concentration in mainland China has been calculated based on satellite data, and it has been found that the majority of the country exceeds the World Health Organization limits of 10 $\mu$g/m$^3$, with some regions reaching and even exceeding PM2.5 concentrations of 100 $\mu$g/m$^3$.

Standard reference measurement methods are based on measuring the mass of deposited or captured particles per air sampling volume for example using a quartz crystal microbalance, a tapered resonator, an impactor, or weighing filters and sieves. There is also a desire to detect specific chemicals within the air, in addition to (or instead of) measuring particle concentrations.

However, known systems often require professional operational guidelines for handling the manual part of the measurement (e.g. weighing a filter and sieve) and/or periodic maintenance for cleaning the accumulated mass, maintaining various system components and calibration.

It has been recognized that there is a need for lower cost and simpler to use sensing technologies, so that complicated and expensive scientific instruments are not needed.

Generally, low cost sensors which can for example be applied in air purifiers for consumer use have reduced performance in sensitivity and reliability compared to professional range sensors which are much more expensive. Many low cost sensors only respond to higher pollutant concentrations than is desired. It may also be that their response is not linear in the low concentration region.

For many sensors, the operation principles result in a response to other compounds than the target compound, leading to incorrect readings when a target compound and an interfering compound are present simultaneously.

Taking an electrochemical formaldehyde sensor as example, compounds like alcohols and detergents can greatly influence the output, and these other compounds are commonly seen in real home conditions. Sensor contamination is another challenge in most real life applications. After being exposed to dirty environments for a period of time, sensors tend to become contaminated by substances like oily particles or poisoning gases, resulting in output decay. A temperature change of the environment also often causes shift of sensor response. Incorrect readings are almost inevitable and sensors are unreliable if no (re) calibration is performed.

It is known to use pre-filters upstream from the sensor, to protect the sensor from contamination and interfering gases.

WO 2013/133872 discloses the use of a honeycomb filter to screen out interfering compounds that lead to false readings of indoor formaldehyde gas or other target gas. This is effective in the case that the interfering substances are of very low concentration. However, for sensors used in air purifiers or other appliances, high concentration interfering and/or poisoning substances like detergents, alcohols and oily aerosol, which are often emitted in daily life (for example during a party or during cooking), can saturate the pre-filters quickly and again leave the sensors unreliable.

Thus, conventional sensing systems, for example used in an air purifier, use one sensor and become unreliable after the sensor runs for a period of time. The problem can be partly alleviated by using pre-filtering, but the efficacy of such a counter measure is still restricted. As the pre-filter has to keep working with the sensor in a single-sensor system, its useful operational lifetime is soon reached particularly when the pollutant concentration is high. This condition also cannot be detected easily.

It is known for co-operating sensing systems to use sensors of different types to do inter-calibration. For example, US 5 394 934 discloses the use of a VOC (volatile organic compound) sensor and a CO2 sensor, and one sensor is used to modify the reading from the other.

Since the responses of different sensors to interfering substances are not the same, incorrect baseline calibration can result when performing inter-calibration. This means system stability can be deteriorated.

EP0764331A1 discloses a method of recalibrating moisture sensor by having measuring sensor and a calibration sensor. In that system, the sensors are exposed to incoming air directly, and the moisture sensors do not require e.g. filtering before sensing.

EP2762877A1 discloses a system for calibrating chemical sensors by comparing readings from two sensors when it's detected that they are close to each other. However, the publication is not relating to treating the air before exposing the chemical sensors to it, which could lead to a shorter sensor lifetime, and/or interference.

SUMMARY OF THE INVENTION

The invention alleviating at least some of the shortcomings of the known systems is defined by the claims.

According to a first aspect of the invention a sensor system for sensing a substance in a fluid, as set forth in claim 1. The fluid may be an aerosol such as air or any other gas with entrained particles or contaminant gases. In other embodiments, the fluid may also be liquid such as water, water solution of chemicals, or the like.

Preferably, a duty cycle of operation of the second sensor is lower than a duty cycle of operation of the first sensor. In this way, the second sensor ages at a slower rate so that it can be used to calibrate the more rapidly ageing first sensor.

This sensor system enables prolonged (such as continuous) monitoring using a first sensor, which may be a low cost sensor rather than an expensive scientific instrument (although even expensive scientific equipment may need regular calibration). Drift and/or nonlinearity in the output signal from the first sensor can be compensated by the second sensor, which is of the same type as the first, but operated with a lower duty cycle. The second sensor performs discrete calibration measurements, which can then be used to adjust the way the output signals from the first sensor are interpreted. It is also possible to indicate an expiration of the lifetime of the first sensor by processing the output signals from both the first sensor and the second sensor, in case the drift and/or nonlinearity in the output signal from the first sensor is found severe enough, and it is impossible to calibrate the first sensor.

By using the second sensor with a lower duty cycle, the second sensor is subject to output drift or other ageing effects more slowly than the first, so it can be used to compensate for such drift using a calibration approach. The first sensor may stop operating during the calibration events, or it may continue to monitor. The two sensors use the same sensing methodology (e.g. mass sensing, optical scattering detection, optical transmission detection, electrical charge measured after charging the particles, or chemical sensing, etc. Note that this list is not complete and not meant to restrict the sensing principles used. The sensors are also configured for detection of the same substance, (e.g. the same chemical species or the same particle size range). The term "substance" should thus be understood to relate to a specific chemical or class of chemicals, or a specific particle size or range of particle sizes, including also microorganisms, viruses, spores and the like. Again this list is not complete and not meant to limit the invention.

The first and second sensors can be identical, or they may be scaled equivalents. By having the two sensors of the same type, the calibration operation is as effective as possible. The sensors have the same response to the prevailing conditions, so that background correlation or subtraction can be carried out effectively.

The calibration events may simply be periodically carried out, or else the timing may be controlled based on information which is indicative of when a calibration may be needed. The sensing history of the first sensor may for example be used to determine when a calibration should be carried out for example based on how high the concentration of the target substance has been.

The calibration may comprise adjusting the zero point and/or the sensitivity. The sensitivity may include for example the linearity of the sensor response such as the gradient of a linear sensor response function. The sensitivity correction may for example also include adjusting the light intensity of LEDs or laser diodes used in optical sensors.

At the end of the life of the first sensor, the second sensor can be used in its place, and a new calibration sensor can be installed.

The sensor system may be for measuring a pollutant amount or concentration in air, or sensing a target substance in a fluid such as air.

In one example, the first and second sensors each comprise a mechanical sensor. The mechanical sensors may each comprise a sensing element, and a transducer adapted to drive the sensing element into resonance and to detect a resonance frequency of the sensing element, wherein the resonance frequency is dependent on a mass of particles deposited on the sensing element.

The mechanical sensor in this case is a resonant mass sensor which detects changes in resonance frequency. This may for example comprise a MEMS (micro electro mechanical system) sensor.

In another example, the first and second sensors each comprise a light scattering optical sensor.

This may for example comprise a nephelometer. This is a readily available component. Alternatively, a specifically designed optical unit may be used.

In another example, the first and second sensors each comprise a gas sensor. Each gas sensor may comprise an electrochemical sensor or a MOX-based (metal oxide semiconductor) sensor.

The sensor system may further comprise a sample intake device for driving the fluid monitored towards the sensor being used.

For an aerosol, the sample intake device may comprise a fan or an electrostatic attraction arrangement, or else a thermophoretic or gravitational based system may be used. In this way, the second sensor may not be exposed to the target while it is not being used. The operation of the sensor may instead provide the sample intake function, for example by electrostatic attraction, so that when the sensor is not being used it is not exposed to the target.

The sensor system also comprises a first filter coupled to the first sensor and a second filter coupled to the second sensor.

These filters can carry out pre-filtering to enable the lifetime of the sensors to be extended.

The first filter may be for selecting a range of particle sizes from air for supply to the first sensor and the second filter may be for selecting a range of particle sizes from air for supply to the second sensor. This means that for particle analysis, the sensing is carried out only for a range of particle sizes of interest. For example, the filtration arrangement may ensure that large particles are prevented from reaching the sensors. For example a filtration arrangement may capture particles greater than a size threshold such as $2.5\mu m$, for PM2.5 measurement.

The system may further comprise a valve arrangement for selectively routing fluid filtered by the first and second filters to a selected one of the first and second sensors.

This can be used as a way to diagnose when the first filter (which is used for longer overall time than the second filter) needs to be changed, based on comparing the signals received by one of the sensors (preferably the second sensor) when receiving filtered fluid from the two filters in turn.

An air treatment device can use the sensor system of the first aspect of the invention. According to a second aspect of the invention, a method for sensing a substance in a fluid is specified in claim 12. The invention can thus be used for providing calibration of a range of different sensor types. This may be used to improve the performance of low cost sensors, but equally the method may be applied to higher cost sensors.

The method comprises filtering the fluid before supply to the first sensor using a first filter and filtering the fluid before supply to the second sensor using a second filter, wherein the method further comprises:

performing a test of the first filter by routing fluid filtered by the first and second filters to one of the first and second sensors in turn, and determining a filter status from the sensor measurements.

This enables the filter status to be determined automatically.

BRIEF DESCRIPTION OF THE DRAWINGS

Examples of the invention will now be described in detail with reference to the accompanying drawings, in which:

Fig. 1 shows a first example of sensor system and associated timing diagram;

Fig. 2 shows a method of operating the sensor system of Fig. 1;

Fig. 3 shows a second example of sensor system;

Fig. 4 shows a method of operating the sensor system of Fig. 3; and

Fig. 5 shows how sensor response characteristics can change over time;

Fig. 6 shows measurements which can be taken for a baseline calibration;

Fig. 7 is used to explain a first approach for sensitivity calibration;

Fig. 8 is used to explain a second approach for sensitivity calibration; and

Fig. 9 shows one example of possible sensor structure.

DETAILED DESCRIPTION OF THE EMBODIMENTS

The invention provides a sensor system for sensing a substance in a fluid, comprising first and second sensors of the same type. The first sensor is used until a calibration event, during which the second sensor is used. The second sensor is used only during the calibration event such that a duty cycle of operation of the second sensor is lower than the duty cycle of operation of the first sensor. The first sensor is recalibrated using the sensor information from the second sensor.

In this way, two sensors of the same kind are used in the sensing system. One working sensor operates in conventional manner for continuous sensing. The other, calibration, sensor works intermittently to perform real time internal calibration. Periodic calibration overcomes the reliability problem of the working sensor in long term operation. The calibration sensor (and any associated pre-filter) is only used for a short time, guaranteeing the availability of a long-term service as well. By using sensors of the same kind, responses from interfering substances can be balanced out.

The sensing thus has at least two modes: a sensing mode and a calibration mode. Signal subtraction between the two sensors can be used to eliminate interference from other substances during calibration. The time of calibration is shorter, and preferably much shorter, than the overall sensing time. The ratio between sensing time and calibration time may for example be tunable.

A diagnostic mode may also be implemented, as discussed below.

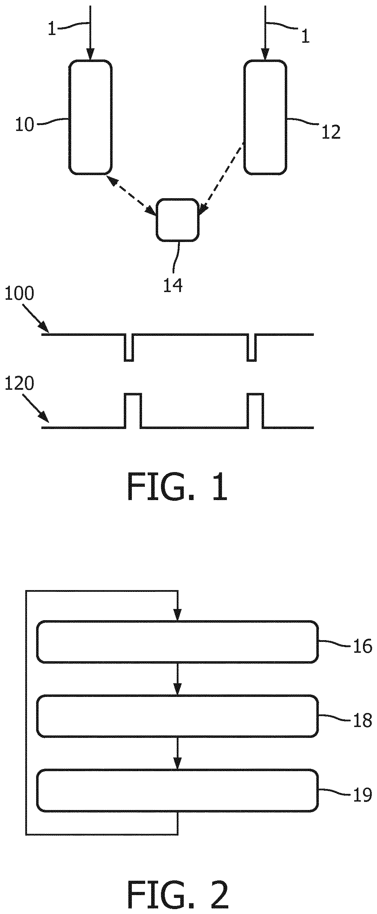

Fig. 1 shows a first example of a sensor system for sensing a substance in a fluid 1. The fluid may be an air stream which contains particulate matter to be sensed, or the sensing may be for detecting and/or measuring the quantity of a target pollutant. The "substance" to be sensed may thus comprise a known chemical or category of chemical or any particulate matter (which may be of known or unknown chemical composition).

The system comprises a first sensor 10 for sensing the substance in the fluid 1 and a second sensor 12 for sensing the substance in the fluid 1, the second sensor 12 being of the same type as the first sensor 10 and for sensing the same substance.

A controller 14 is adapted to:

sense the substance in the fluid using the first sensor 10, until a calibration event;
during the calibration event, sense the substance in the fluid using the second sensor 12, the second sensor being operated only during the calibration event such that a duty cycle of operation of the second sensor is lower than the duty cycle of operation of the first sensor; and
recalibrate the first sensor using the sensor information from the second sensor.

The first sensor 10 may provide continuous monitoring and/or measurement. Drift and/or sensitivity changes in the output signal from the first sensor 10 can be compensated by the second sensor during a discrete calibration measurement. The first sensor 10 may stop operating during the calibration events, or it may continue to monitor.

In the example of Fig. 1, two cheap sensors can be used without pre-filters. The first sensor 10 functions as a working sensor and the second sensor 12 functions as a calibration sensor.

The air stream 1 can pass through both sensors, and it can be original air from the targeted environment, or else pre-filters can be used upstream. The controller 14 retrieves signals from both sensors and performs the calibration of the first sensor 10.

The timing diagram in Fig. 1 represents the timing for the operation of the sensors 10 and 12 as plots 100 and 120 respectively. Most of time, the second sensor 12 is separated from the air stream and sensor 10 is exposed to the air stream for sensing. Periodically, the second sensor 12 is turned on and exposed to the air stream 1 for calibration. After a short while, sensor 12 is turned off and separated from air stream again until the next cycle. The first sensor 10 is shown as deactivated during the calibration, but it may continue to operate, since the calibration is a signal processing modification rather than a modification to a physical configuration of the first sensor 12.

The difference between the signals as received from the two sensors 10, 12 can be used for baseline adjustment of the first sensor. The baseline adjustment comprises adjusting the zero point. However, the sensitivity may also be recalibrated and can be implemented by changing a calibration factor in the software used in operating the sensor.

Fig. 2 shows the method for using the sensor system of Fig. 1. In step 16 the sensor 10 is used to sense the substance in the fluid 1 (e.g. air stream) until a calibration event. During the calibration event, the sensing takes place with the second sensor 12 in step 18. The second sensor is operated only during the calibration event, i.e. in step 18, such that a duty cycle of operation of the second sensor is lower than a duty cycle of operation of the first sensor. Step 19 is the calibration of the first sensor.

The two sensors use the same sensing methodology. There are various possible types of sensor, and a non-exhaustive list is:

a mechanical sensor;
an optical sensor;
a chemical sensor, for example an electrochemical sensor or a MOX sensor;
an electrostatic sensor;
a sensor based on detecting differences in thermal behavior;
a flame ionization detector.

The two sensors are also configured for detection of the same substance, (e.g. the same chemical species or the same particle size range). The first and second sensors can be identical, but they may be scaled equivalents so that their output signals still respond in the same way to the target and to pollutants.

The calibration events may simply be periodically carried out. A long delay between calibration events will give a longer lifetime of the calibrating sensor but will result in more signal drift between the calibration events. The frequency of the calibration may for example by set by the user.

The timing of calibration events may instead be controlled based on information which is indicative of when a calibration may be needed. The sensing history of the first sensor may for example be used to determine when a calibration should be carried out.

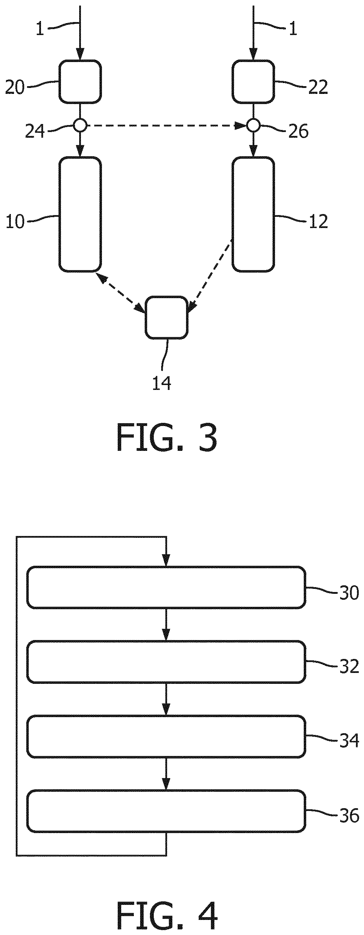

Fig. 3 shows an embodiment of the invention in which the first sensor 10 has a pre-filter 20 and the second sensor 12 has a pre-filter 22. The pre-filters are used to remove known expected pollutants, or to limit the particle size range reaching the sensors within a defined.

The output from the first pre-filter 20 is supplied to a first valve 24 which can supply the pre-filtered air to either one of the sensors 10,12. The output from the second pre-filter 22 is supplied to a second valve 26 which can allow or block the pre-filtered air passing to the second sensor 12.

The valve arrangement enables online diagnosis of the pre-filters as well as the deterioration of the performance of the working sensor 10. For the diagnosis functions, three series of data are used:

(i) the response signals of the working sensor 10 to working air,
(ii) the response signals of the calibration sensor 12 to working air (i.e. from the filter 20),
(iii) the response signals of the calibration sensor 12 to the calibration air (i.e. from the filter 22)

The working air from the pre-filter 20 may contain undesired pollutants if the pre-filter 20 fails. The calibration air from the pre-filter 22 is assumed to be free of the pollutant as a result of the low duty cycle with which the pre-filter 22 is used.

Fig. 4 shows the method.

In step 30 the working sensor 10 is used to sense air from the first pre-filter 20.

In step 32, the calibration sensor 12 is used to sense air from the first pre-filter 20. In step 34, the calibration sensor 12 is used to sense air from the second pre-filter 22. Steps 32 and 34 may be considered together to comprise part of a calibration event, and the duration of step 30 is again longer than the duration of steps 32 and 34.

If the signal in step 32 is very different from that in step 34 for the first time, it means the pre-filter 20 is running out, because the sensor response to the air flow filtered by the two pre-filters is very different.

The difference between the signals from steps 30 and 32 indicates the deterioration or baseline shift of the sensor 10, because the two sensors have different responses to the same air supply.

Before the pre-filter 20 is changed, the information gathered about the filter status can be used to modify the interpretation of the data from the first sensor 10. Thus, in the final processing step 36, the system can determine when the filter 20 needs to be changed, it can perform baseline correction based on the different sensor responses to the same stimulus, and it can perform linearity and stability adjustments to the signal processing based on the filter performance. The linearity of the working sensor can for example be recalibrated by correlating the trend lines of the two sensors.

The signal produced by each sensor comprises a value generated in the absence of a pollutant (the baseline) to which a value is added that depends on the pollutant concentration. The simplest correction is based on assuming that the baseline of the two sensors has not changed. Any change in the signal can then be corrected by changing a calibration factor (with which the sensor signal is multiplied). This is in general not sufficient as a full calibration operation, in which case the more complete calibration operations described below can be used.

There are many types of sensor which, when operated without fluid (i.e. air) flowing through, produce a signal that does not contain any pollutant information. This then gives a baseline signal. This applies to all the sensor principles mentioned above. In the case of such sensors, baseline differences are easily detected and corrected, for example in the software used to operate the sensor.

When operating the sensors with polluted air, differences in sensitivity, rather than baseline, can be detected and corrected in a similar manner, using the signals produced by the sensors. For optical sensors based on light scattering, the intensity of the light source can also be adjusted, within limits inherent to the operation principle of the light source. Alternatively, also, within limits inherent to the operation principle, the voltage over the photodetector can be adjusted. Similar measures can also be taken in case of sensors based on optical absorption.

One baseline calibration method has been described above. Some further possible calibration steps will now be discussed.

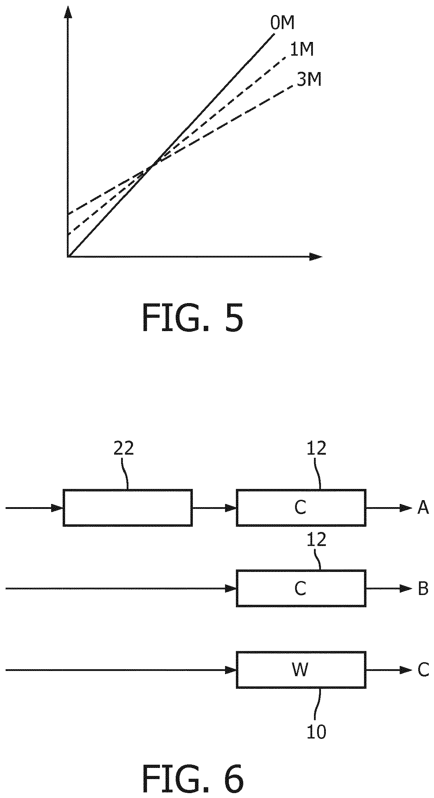

Fig. 5 shows how a linear sensor response (as concentration versus signal recorded) may vary over time for a new sensor (plot 0M), for a one month old sensor (plot 1M) and for a three month old sensor (plot 3M). The gradient changes, which is illustrative of the sensitivity, and the zero point at which there is no signal also changes.

Both of these properties of the sensor response should be recalibrated over time. As the sensor deteriorates, it may only be possible to obtain good signals at very high concentrations. In such a case, for some optical sensors, it is also possible to control the working conditions of the sensor (for example increase the power of the light source) to compensate for the loss of sensitivity.

Fig. 6 is used to illustrate another baseline calibration approach. Three signal measurements are made. Signal A is the response of the calibration sensor 12 to an input which has been pre-filtered by filter 22, which functions to remove the target substances of interest. Signal B is the response of the calibration sensor 12 to an input which has not been pre-filtered. Signal C is the response the working sensor 10 to an input which has not been pre-filtered and thus includes the target substances of interest.

The clean calibration sensor signal A can be used as a zero point baseline signal, and can thus be used to interpret the working sensor signal C. This approach assumes that the two sensors have similar baseline values, since the baseline is obtained using the calibration sensor, and it is then assumed to apply also to the working sensor. Signals B and C are the normal working and calibration sensor signals as used for the calibration process described above.

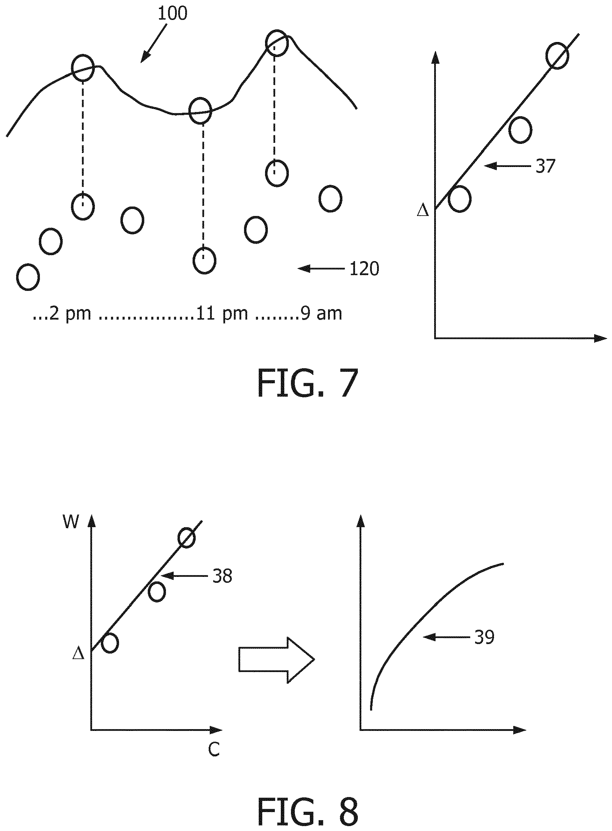

For a zero point and sensitivity calibration, a first approach is explained with reference to Fig. 7.

A number of calibration points are taken at different pollutant levels, for example three or five. Plot 100 is the working sensor signals and plot 120 is the calibration sensor signals. The requirement for different pollutant levels is generally met in daily life. This enables the sensor response curve to be formed as the line of best fit as shown as plot 37. This again shows the concentration versus signal recorded. The correct zero point and slope of the line can be calculated, which are used later to calculate other measurement results. When very poor linearity is found in the calibration, it means the working sensor is at the end of its life.

This assumes a linear sensor response.

If the kind of sensor used in principle has a non-linear response to different pollutant concentrations, an alternative approach is explained with reference to Fig. 8.

The correlation between the two sensors is first found, as shown in plot 38. This plots the working sensor signal W against the calibration sensor signal C. The working sensor function shown as plot 39 can be obtained by interpolation, with the working sensor response being calculated as a converted version of the calibration sensor response, which is assumed to be static. The plot 39 is again the concentration versus signal recorded.

If poor linearity is found between the two sensors, it means the working sensor is at the end of its life.

At the end of the life of the first sensor 10, for example detected as explained above, the second sensor 12 can be used in its place, and a new calibration sensor can be installed. In this way, the calibration sensor is always near the beginning of its lifetime.

Note that the simplest implementation of Fig. 1 does not use valves to allow air paths to be swapped. In that case, the diagnosis is based only on two measurements, one by each sensor of the same incoming air supply.

As mentioned above, the invention can be applied to different types of sensor.

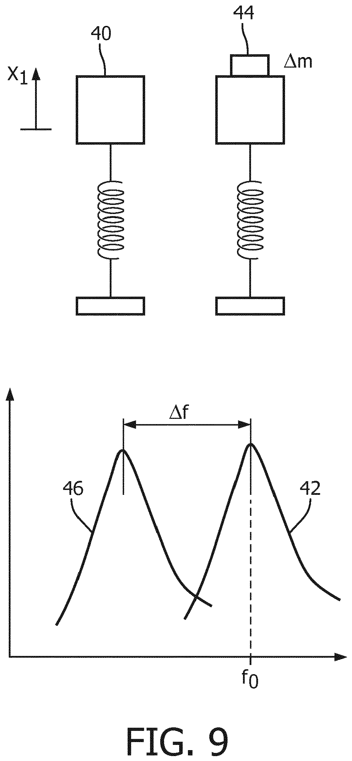

One example is a mass sensor, for example for measuring the particle concentration of pollutants. Direct mass measurement using resonant devices is a known technique. It is based on the relationship between the resonance frequency ($f_0$) and the mass of a resonator, as shown in Fig. 9.

In Fig. 9 a resonator mass 40 is represented schematically, with a mass m and spring constant k. The graph shows the amplitude of the resonant oscillations (on the y-axis) as a function of frequency (the x-axis). Plot 42 is for the basic resonator mass. If an additional mass 44 is added ($\Delta m$), the oscillation curve shifts down in frequency to plot 46 with a frequency shift $\Delta f$.

The equations which govern the resonant vibrations are;

$$f_0 = \frac{1}{2\pi}\sqrt{\frac{k}{m}} \qquad (1)$$

$$\Delta f = -\frac{1}{2}\frac{\Delta m}{m}f_0 \qquad (2)$$

$$\Delta m_{min} \propto \frac{m}{Q} \qquad (3)$$

Equation (1) shows the relationship between the basic resonance frequency and the resonator characteristics. Equation (2) shows the change in frequency caused by a change in mass, and equation (3) shows the minimum mass ($\Delta m_{min}$) that can be detected. The minimum depends on the mechanical quality factor Q of the resonator.

There are several examples of resonance based mass sensing for aerosol contamination monitoring in literature. For example, use of a micromachined silicon cantilever device with a picogram level of mass resolution for personal exposure monitoring has been proposed. Filters can be used for eliminating large particles and an electrostatic sampler can be provided for depositing nanoparticles on the cantilever.

As a rule of thumb, mechanical sensors which operate by monitoring changes in resonance frequency operate in a range where the added mass is small compared to the initial resonator mass. However, continuous mass accumulation during the lifetime of the sensor is inevitable. This problem is more pronounced for MEMS scale devices, in which mechanical and/or chemical cleaning of the accumulated mass is not possible - at least for consumer applications. Therefore, the lifetime of a MEMS sensor can be roughly estimated by considering the initial mass and the approximate mass deposition per measurement cycle.

Optical sensing technologies (for PM2.5) have also been proposed for consumer level applications for air purifiers. The primary technology for consumer level applications is based on the optical scattering by suspended particles in air (e.g., nephelometry). The sensor accuracy depends on the quality of the optical pathway, for example depending on the presence and position of pollutant particles. Nephelometers use a light source and an optical detector. The method is essentially based on measuring the scattered light intensity by suspended particles in air (or other carrier gas).

The optical sensor measurement is based on the scattered light, in addition to other factors. The performance of this kind of sensor thus depends on the intensity of the scattered light. Over time, the brightness of the light source used by the optical sensor (for example an LED) decreases, and this gives rise to sensor drift. There is also an issue with contamination, which is more pronounced for indoor aerosols, especially oily aerosols which may form a layer on various optical components of the system (lenses, LED surface, etc.). This results in a decrease in the light intensity, hence false measurements.

The calibration enabled by the system addresses issues with long term sensor signal drift or non-linearity. The invention can be applied to the optical or mechanical sensors outlined above, but also to chemical sensors (for example electrochemical sensors), electrostatic sensors, sensors based on detecting differences in thermal behavior or flame ionization detectors.

To avoid the calibration sensor ageing at the same rate as the working sensor, it should not be exposed to the pollutant when not in use. The supply of the fluid being analyzed can be controlled by electrostatic or electrophoretic precipitation in the case of charged particles, on a grounded or oppositely biased resonator. Thermophoretic precipitation may instead be used which comprises creating a temperature difference between the sensor and a counter surface. A fan or pump for delivering a sampled fluid volume may also be used.

The sensor system may be for measuring a pollutant amount or concentration in air, or for sensing a target substance in a fluid such as air.

The duty cycle may be 0.1 or less, i.e. the calibration sensor is used for 10% or less of the time that the working sensor is used. This also means the calibration sensor is always at the beginning 10% of its lifetime, if it is then used to replace an expired working sensor and replaced by a new calibration sensor.

The calibration may be performed daily, weekly, or only after longer periods such as several weeks or months. The duty cycle may thus be extremely low so the calibration sensor is operated only for a very short period of time.

Applications of interest include air purifiers, stand-alone particle sensor units, personal exposure monitoring devices, vehicle cabin particle measurement sensors, particle sensors for outdoor use (as a standalone sensor unit or for example, sensors for lamp posts for city management), ventilation units, various parts of a building climate management system and in general various types of mechanical sensor which operate by detecting changes in resonance frequency. There are also medical applications in respiratory support and drug delivery applications.

The system makes use of a controller. Components that may be employed for the controller include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller.

**Claims**

1. A sensor system for determining the status of a filter, comprising:

> a first sensor (10) for sensing the substance in the fluid;
> a second sensor (12) for sensing the substance in the fluid, the second sensor (12) being of the same type as the first sensor (10) and for sensing the same substance; and
> a controller (14) adapted to:
>
> > control the first sensor (10) to sense the substance in the fluid, until a calibration event; during the calibration event, control the second sensor (12) to sense the substance in the fluid, the second sensor (12) being operated only during the calibration event; calibrate the first sensor (10) using the sensor information from the second sensor (12);

> **characterised by** further comprising:

> > a first filter (20) coupled to the first sensor (10) and a second filter (22) coupled to the second sensor (12); wherein the sensor system is configured to route fluid filtered by the first and second filters (20, 22) to one of the first and second sensors (10, 12) in turn, and to determine a status of the first filter (20) based on a difference between signals from the first and the second sensor (10, 12).

2. A sensor system as claimed in claim 1, wherein the sensor system is configured for sensing the presence and/or amount of a target substance in a fluid.

3. A sensor system as claimed in claim 2, wherein the sensor system is configured for sensing a pollutant amount or concentration in air.

4. A sensor system as claimed in claim 1, 2 or 3, wherein the first and second sensors (10,12) each comprise a mechanical sensor.

5. A sensor system as claimed in claim 4, wherein the mechanical sensors each comprise a sensing element, and a transducer adapted to drive the sensing element into resonance and to detect a resonance frequency of the sensing element, wherein the resonance frequency is dependent on a mass of particles deposited on the sensing element.

6. A sensor system as claimed in claim 1, 2 or 3, wherein the first and second sensors (10,12) each comprise a light scattering optical sensor.

7. A sensor system as claimed in claim 1, 2 or 3, wherein the first and second sensors (10,12) each comprise a gas sensor, or such as an electrochemical sensor or a MOX-based gas sensor.

8. A sensor system as claimed in any one of claims 1 to 7, further comprising a sample intake device for driving the fluid monitored towards the sensor being used.

9. A sensor system as claimed in claim 1, wherein the first and second sensors (10, 12) are particle sensors, the first filter (20) is for selecting a range of particle sizes from air for supply to the first sensor (10) and the second filter (22) is for selecting a range of particle sizes from air for supply to the second sensor (12).

10. A sensor system as claimed in claim 1 or 9, further comprising a valve arrangement (24,26) for selectively routing fluid filtered by the first and second filters (20, 22) to a selected one of the first and second sensors (10, 12).

11. An air treatment device, comprising a sensor system as claimed in any one of claims 1 to 10.

12. A sensing method for determining the status of a filter, comprising:

sensing (16;30) a substance in a fluid using a first sensor (10) until a calibration event; during the calibration event, sensing (18;32) the substance using a second sensor (12), the second sensor (12) being of the same type as the first sensor (10) and for sensing the same substance, the second sensor (12) being operated only during the calibration event; and calibrating (19;36) the first sensor (10) using the sensor information from the second sensor (12); and filtering the fluid before supply to the first sensor (10) using a first filter (20) and filtering the fluid before supply to the second sensor (12) using a second filter (22),

characterised in that the method further comprises:

performing (32,34) a test of the first filter (20) by routing fluid filtered by the first and second filters (20, 22) to one of the first and second sensors (10, 12) in turn, and determining (36) a first filter (20) status based on a difference between signals from the first and the second sensors (10, 12).

13. A method as claimed in claim 12, wherein the first and second sensors (10,12) each comprise:

a physical sensor, such as a mechanical, optical, thermal, acoustic, electrostatic, or electromagnetic sensor; or a chemical sensor, such as an electrochemical sensor or a MOX sensor; or a hybrid sensor, such as a flame ionization detector.

**Patentansprüche**

1. Sensorsystem zum Bestimmen des Status eines Filters, umfassend:

einen ersten Sensor (10) zum Messen der Substanz in dem Fluid; einen zweiten Sensor (12) zum Messen der Substanz in dem Fluid, wobei der zweite Sensor (12) vom gleichen Typ wie der erste Sensor (10) und zum Messen der gleichen Substanz ist; und eine Steuerung (14), die angepasst ist zum:

Steuern des ersten Sensors (10), um die Substanz in dem Fluid bis zu einem Kalibrierungsereignis zu messen; Steuern des zweiten Sensors (12) während des Kalibrierungsereignisses, um die Substanz in dem Fluid zu messen, wobei der zweite Sensor (12) nur während des Kalibrierungsereignisses betrieben wird; Kalibrieren des ersten Sensors (10) unter Verwendung der Sensorinformation von dem zweiten Sensor (12); **dadurch gekennzeichnet, dass** es weiterhin umfasst:

einen ersten Filter (20), der mit dem ersten Sensor (10) gekoppelt ist, und einen zweiten Filter (22), der mit dem zweiten Sensor (12) gekoppelt ist; wobei das Sensorsystem konfiguriert ist, durch den ersten und den zweiten Filter (20, 22) gefiltertes Fluid der Reihe nach zu einem der ersten und zweiten Sensoren (10, 12) zu leiten und basierend auf einer Differenz zwischen Signalen von dem ersten und dem zweiten Sensor (10, 12) einen Status des ersten Filters (20) zu bestimmen.

2. Sensorsystem nach Anspruch 1, wobei das Sensorsystem zum Messen des Vorhandenseins und / oder der Menge einer Zielsubstanz in einem Fluid konfiguriert ist.

3. Sensorsystem nach Anspruch 2, wobei das Sensorsystem zum Messen einer Schadstoffmenge oder -konzentration in Luft konfiguriert ist.

4. Sensorsystem nach Anspruch 1, 2 oder 3, wobei der erste und der zweite Sensor (10, 12) jeweils einen mechanischen Sensor umfassen.

5. Sensorsystem nach Anspruch 4, wobei die mechanischen Sensoren jeweils ein Messelement und einen Wandler umfassen, der angepasst ist, um das Messelement in Resonanz zu treiben und eine Resonanzfrequenz des Messelements zu messen, wobei die Resonanzfrequenz von einer Masse von auf dem Messelement abgeschiedenen Partikeln abhängt.

6. Sensorsystem nach Anspruch 1, 2 oder 3, wobei der erste und der zweite Sensor (10, 12) jeweils einen optischen Lichtstreuungssensor umfassen.

7. Sensorsystem nach Anspruch 1, 2 oder 3, wobei der erste und der zweite Sensor (10, 12) jeweils einen Gassensor oder beispielsweise einen elektrochemischen Sensor oder einen MOX-basierten Gassensor umfassen.

8. Sensorsystem nach einem der Ansprüche 1 bis 7, weiterhin umfassend eine Probenaufnahmevorrichtung zum Antreiben des überwachten Fluids zu dem verwendeten Sensor.

9. Sensorsystem nach Anspruch 1, wobei der erste und der zweite Sensor (10, 12) Partikelsensoren sind, wobei der erste Filter (20) zum Auswählen eines Bereichs von Partikelgrößen aus Luft zum Zuführen zu dem ersten Sensor (10) ist und der zweite Filter (22) zum Auswählen eines Bereichs von Partikelgrößen aus Luft zum Zuführen zu dem zweiten Sensor (12) ist.

10. Sensorsystem nach Anspruch 1 oder 9, weiterhin umfassend eine Ventilanordnung (24, 26) zum selektiven Leiten von durch den ersten und zweiten Filter (20, 22) gefiltertem Fluid zu einem Ausgewählten der ersten und zweiten Sensoren (10, 12).

11. Luftbehandlungsvorrichtung, umfassend ein Sensorsystem nach einem der Ansprüche 1 bis 10.

12. Messverfahren zum Bestimmen des Status eines Filters, umfassend:

Messen (16; 30) einer Substanz in einem Fluid unter Verwendung eines ersten Sensors (10) bis zu einem Kalibrierungsereignis; während des Kalibrierungsereignisses Messen (18; 32) der Substanz unter Verwendung eines zweiten Sensors (12), wobei der zweite Sensor (12) vom gleichen Typ wie der erste Sensor (10) ist und zum Messen der gleichen Substanz wobei der zweite Sensor (12) nur während des Kalibrierungsereignisses betrieben wird; und Kalibrieren (19; 36) des ersten Sensors (10) unter Verwendung der Sensorinformation von dem zweiten Sensor (12); und Filtern des Fluids vor dem Zuführen zu dem ersten Sensor (10) unter Verwendung eines ersten Filters (20) und Filtern des Fluids vor der Zuführ-

rung zu dem zweiten Sensor (12) unter Verwendung eines zweiten Filters (22), **dadurch gekennzeichnet, dass** das Verfahren weiterhin umfasst:

Durchführen (32, 34) eines Tests des ersten Filters (20) durch Leiten von durch den ersten und den zweiten Filter (20, 22) gefiltertem Fluid zu einem der ersten und zweiten Sensoren (10, 12) der Reihe nach, und Bestimmen (36) eines Zustands eines ersten Filters (20) basierend auf einer Differenz zwischen Signalen von dem ersten und dem zweiten Sensor (10, 12).

13. Verfahren nach Anspruch 12, wobei der erste und der zweite Sensor (10, 12) jeweils umfassen:

einen physikalischen Sensor, wie einen mechanischen, optischen, thermischen, akustischen, elektrostatischen oder elektromagnetischen Sensor; oder einen chemischen Sensor, wie beispielsweise einen elektrochemischen Sensor oder einen MOX-Sensor; oder einen Hybridsensor, wie einen Flammenionisationsdetektor.

## Revendications

1. Système de capteurs pour déterminer le statut d'un filtre, comprenant :

un premier capteur (10) pour détecter la substance dans le fluide ; un second capteur (12) pour détecter la substance dans le fluide, le second capteur (12) étant du même type que le premier capteur (10) et servant à détecter la même substance ; et

un dispositif de commande (14) adapté pour :

commander au premier capteur (10) de détecter la substance dans le fluide, jusqu'à un évènement d'étalonnage ; pendant l'évènement d'étalonnage, commander au second capteur (12) de détecter la substance dans le fluide, le second capteur (12) étant mis en fonctionnement uniquement pendant l'évènement d'étalonnage ; étalonner le premier capteur (10) à l'aide des informations de capteur issues du second capteur (12) ; **caractérisé en ce qu'**il comprend en outre :

un premier filtre (20) couplé au premier capteur (10) et un second filtre (22) couplé au second capteur (12) ;

dans lequel le système de capteurs est configuré pour acheminer le fluide filtré par les premier et second filtres (20, 22) vers l'un des premier et second capteurs (10, 12) tour à tour, et pour déterminer un statut du premier filtre (20) d'après une différence entre des signaux issus du premier et du second capteur (10, 12).

2. Système de capteurs selon la revendication 1, dans lequel le système de capteurs est configuré pour détecter la présence et/ou la quantité d'une substance cible dans un fluide.

3. Système de capteurs selon la revendication 2, dans lequel le système de capteurs est configuré pour détecter une quantité ou une concentration de polluant dans l'air.

4. Système de capteurs selon la revendication 1, 2 ou 3, dans lequel les premier et second capteurs (10, 12) comprennent chacun un capteur mécanique.

5. Système de capteurs selon la revendication 4, dans lequel les capteurs mécaniques comprennent chacun un élément de détection, et un transducteur adapté pour piloter l'élément de détection en résonance et pour détecter une fréquence de résonance de l'élément de détection, dans lequel la fréquence de résonance dépend d'une masse de particules déposées sur l'élément de détection.

6. Système de capteurs selon la revendication 1, 2 ou 3, dans lequel les premier et second capteurs (10, 12) comprennent chacun un capteur optique de diffusion de lumière.

7. Système de capteurs selon la revendication 1, 2 ou 3, dans lequel les premier et second capteurs (10, 12) comprennent chacun un capteur de gaz, ou tel qu'un capteur électrochimique ou un capteur de gaz à base de MOX.

8. Système de capteurs selon l'une quelconque des revendications 1 à 7, comprenant en outre un dispositif d'admission d'échantillon pour entraîner le fluide surveillé vers le capteur utilisé.

9. Système de capteurs selon la revendication 1, dans lequel les premier et second capteurs (10, 12) sont des capteurs de particule, le premier filtre (20) sert à sélectionner une plage de tailles de particule de l'air à fournir au premier capteur (10) et le second filtre (22) sert à sélectionner une plage de tailles de particule de l'air à fournir au second capteur (12).

10. Système de capteurs selon la revendication 1 ou 9, comprenant en outre un agencement de vanne (24, 26) pour acheminer sélectivement le fluide filtré par les premier et second filtres (20, 22) vers un capteur sélectionné des premier et second capteurs (10, 12).

11. Dispositif de traitement d'air, comprenant un système de capteurs selon l'une quelconque des revendications 1 à 10.

12. Procédé de détection pour déterminer le statut d'un filtre, comprenant :

la détection (16 ; 30) d'une substance dans un fluide à l'aide d'un premier capteur (10) jusqu'à un évènement d'étalonnage ; pendant l'événement d'étalonnage, la détection (18 ; 32) de la substance à l'aide d'un second capteur (12), le second capteur (12) étant du même type que le premier capteur (10) et servant à détecter la même substance, le second capteur (12) étant mis en fonctionnement uniquement pendant l'évènement d'étalonnage ; et l'étalonnage (19 ; 36) du premier capteur (10) à l'aide des informations de capteur issues du second capteur (12) ; et la filtration du fluide avant fourniture au premier capteur (10) à l'aide d'un premier filtre (20) et la filtration du fluide avant fourniture au second capteur (12) à l'aide d'un second filtre (22), **caractérisé en ce que** le procédé comprend en outre :

la réalisation (32, 34) d'un essai du premier filtre (20) en acheminant le fluide filtré par les premier et second filtres (20, 22) jusqu'à l'un des premier et second capteurs (10, 12) tour à tour, et la détermination (36) d'un statut du premier filtre (20) d'après une différence entre des signaux issus des premier et second capteurs (10, 12).

13. Procédé selon la revendication 12, dans lequel les premier et second capteurs (10, 12) comprennent chacun :

un capteur physique, tel qu'un capteur mécanique, optique, thermique, acoustique, électrostatique, ou électromagnétique ; ou un capteur chimique, tel qu'un capteur électrochimique ou un capteur de MOX ; ou un capteur hybride, tel qu'un détecteur à ionisation de flamme.

FIG. 1

FIG. 2

FIG. 3

FIG. 4

FIG. 5

FIG. 6

FIG. 7

FIG. 8

FIG. 9

**REFERENCES CITED IN THE DESCRIPTION**

**Patent documents cited in the description**

- WO 2013133872 A
- US 5394934 A
- EP 0764331 A1
- EP 2762877 A1